(19) Europäisches Patentamt

European Patent Office

Office européen des brevets

(11) Publication number: **0 203 588 B1**

(12) **EUROPEAN PATENT SPECIFICATION**

(45) Date of publication of patent specification: **27.10.93**

(51) Int. Cl.⁵: **C07B 59/00**, C07C 57/04

(21) Application number: **86107250.2**

(22) Date of filing: **28.05.86**

(54) **Process for the production of deuterated acrylic acid or deuterated methacrylic acid.**

(30) Priority: **29.05.85 JP 113963/85**
**03.06.85 JP 118800/85**

(43) Date of publication of application:
**03.12.86 Bulletin 86/49**

(45) Publication of the grant of the patent:
**27.10.93 Bulletin 93/43**

(84) Designated Contracting States:
**BE DE FR GB NL**

(56) References cited:

**TETRAHEDRON LETTERS, vol. 23, no. 37, 1982, pages 3819-3822, Pergamon Press Ltd., GB; W.J.S. LOCKLEY; "Regioselective deuteration of aromatic and alpha-beta-unsaturated carboxylic acids via rhodium(III) chloride catalysed exchange with deuterium oxide"**

**Tetrahedron Letters, Vol. 15, 1961, pages 516-522**

**Journal of the Chemical Society, Chemical Communications, Nr. 23, 1975, pages 930-932**

(73) Proprietor: **MITSUBISHI RAYON CO., LTD.**
**3-19, Kyobashi 2-chome**
**Chuo-Ku**
**Tokyo 104(JP)**

(72) Inventor: **Kato, Masaaki**
**Mitsubishi Rayon Co., Ltd.**
**20-1, Miyukicho**
**Otake-shi(JP)**
Inventor: **Uno, Tetsuya**
**Mitsubishi Rayon Co., Ltd.**
**20-1, Miyukicho**
**Otake-shi(JP)**
Inventor: **Kobayashi, Masao**
**Mitsubishi Rayon Co., Ltd.**
**20-1, Miyukicho**
**Otake-shi(JP)**
Inventor: **Osuga, Naoto**
**Mitsubishi Rayon Co., Ltd.**
**20-1, Miyukicho**
**Otake-shi(JP)**

(74) Representative: **Lehn, Werner, Dipl.-Ing. et al**
**Hoffmann, Eitle & Partner,**
**Patentanwälte,**
**Postfach 81 04 20**
**D-81904 München (DE)**

**Description**

This invention relates to a process for the production of deuterated acrylic acid or deuterated methacrylic acid.

Although methods for the production of deuterated methacrylic acid have been hardly known, a method for the production of deuterated methyl methacrylate via deuterated acetone cyanohydrin has been proposed by the Journal of Polymer Science 62, S95 (1962). Thus, a method may be thought in which deuterated methacrylic acid is obtained by the hydrolysis of deuterated methacrylamide which is an intermediate in the method. The method consists of preparing deuterated acetone cyanohydrin from deuterated acetone and hydrocyanic acid, treating this with sulfuric acid to form the sulfate of methacrylamide, then hydrolyzing this sulfate with heavy water to give deuterated methacrylic acid. However in this method, the use of deuterated starting materials such as deuterated acetone and heavy water has proven economically unsatisfactory because of the larger number of reaction steps involved. Further, as the related prior art, W.J.S. Lockley has set forth "Regioselective Deuteration of Aromatic and $\alpha,\beta$-unsaturatedcarboxylic acid" in "Tetrahedron Letters" Vol. 23, 3819-3822 (1982) and also "Regioselective Hydrogen Isotope Exchange Labelling of Aromatic and $\alpha,\beta$-Unsaturated Acids" in "Synthesis and Application of Isotopically Labelled Compounds. Proceedings of an International Symposium 1982" on pages 427-428 which was published in 1983. Particularly, concerning $\alpha,\beta$-unsaturated carboxylic acids, it is disclosed there that $\beta$-deuterated $\alpha,\beta$-unsaturated carboxylic acids can be obtained using rhodium (III) chloride as a catalyst. In said references, however, the selective deuterium substitution on the position of hydrogen attached to $\beta$-carbon of double bond is characterized, and it is merely mentioned there that polysubstitution reaction will very slightly occur only in case of crotonic acid and cinnamic acid, but no MAA is referred to there. However, the present invention aims at substitution of hydrogens as many as possible in AA or MAA, with deuteriums, which may be attained effectively and economically in accordance with the method of the present invention. For this reason, an improved method involving fewer steps has been sought.

After extensive research on efficient and practical methods for the production of deuterated methacrylic acid, we discovered a novel manufacturing process involving the direct substitution of hydrogens in methacrylic acid with deuterium, which led us ultimately to the present invention.

According to the present invention there is provided a process for the production of deuterated acrylic acid or deuterated methacrylic acid comprising the direct deuteration of acrylic acid or methacrylic acid with heavy water or a mixture of heavy water and deuterium gas in the presence of a catalyst selected from compound(s) involving palladium, ruthenium, iridium and/or platinum used in an amount of at least 0.023 parts by height per 1 part acid at a temperature up to 300 °C.

As the deuterium source for the substitution of deuteriums for hydrogens in acrylic acid or methacrylic acid, heavy water or mixture of heavy water and deuterium gas may be employed. At least a stoichiometric amount of deuterium with respect to the acrylic acid or methacrylic acid must be present within the reaction system.

Referring to the catalyst, compounds involving palladium, ruthenium, iridium and/or platinum, for example hexachloroiridic acid, tetrakis(triphenylphosphine)-palladium, potassium bromoplatinate, potassium pentachlororuthenate, palladium nitrate and potassium hexahydroxoplatinate are preferred. A mixture of two or more thereof may also be employed.

Referring to another catalyst, compounds involving other platinum group elements may be employed. As compounds involving such platinum group element, there are listed platinum, iridium, palladium or ruthenium per se and, for instance, nitrate, chloride or complex compounds thereof, and more concretely, hexachloroiridic acid, tetrakis(triphenylphosphine)palladium, potassium bromoplatinate, palladium nitrate, potassium hexahydroxoplatinate or potassium tetrachloroplatinate is preferred. Where necessary, these compounds may also be supported on a suitable carrier such as alumina, silica, silicaalumina, diatomaceous earth or active carbon.

The reaction may be conducted either in a gaseous phase or a liquid phase, and under the application of pressure. To inhibit polymerization during the reaction, a suitable polymerization inhibitor such as phenothiazine or hydroquinone may be added as required. Polymerization may also be inhibited by allowing a small amount of oxygen to be present in the reaction mixture.

In the case of carrying out the present invention, acrylic acid or methacrylic acid is allowed to react with the deuterium source, for example heavy water to substitute deuteriums for hydrogens in acrylic acid or methacrylic acid.

Where necessary, the present reaction is carried out in the presence of a solvent which is stable at the reaction temperature, for example dimethylacetamide or dimethylformamide, and at from room temperature to 300°C, but a temperature from 60 to 200°C is preferable, especially preferable from 80 to 150°C, from the standpoint of reaction rate as well as the inhibition of side reactions and polymerization. The reaction time is normally from 20 minutes to 100 hours.

The resulting deuterated acrylic acid or deuterated methacrylic acid which can be obtained in accordance with the present invention may be subject to the esterification reaction with alcohol, for example, methylalcohol or deuterated methylalcohol to obtain deuterated methyl acrylate or deuterated methyl methacrylate, respectively, which are used as materials of low light loss optical plastic fibers. Optical plastic fibers mace of undeuterated methylmethacrylate or undeuterated methyl acrylate are significantly affected by the vibration absorption of the C-H bonds at the light transmission wavelength, so that it is difficult to make light loss fibers. However, the conversion of the C-H bonds to C-D bonds by the deuteration removes the influence of C-H vibration absorption, thereby improving the light transmission ability of fibers. By this reason, the best results may be attained in case that all the C-H bonds have been converted to the C-D bonds, but even in case of a partial deuteration, the effects will be exerted in response to a degree of deuteration.

In the following Examples, all references to "parts" signify parts by weight. Analyses were conducted by means of a gas chromatograph and a mass spectrometer. The deuteration ratio and the conversion ratio are defined as follows:

$$\text{Deuteration ratio} = \frac{\text{Number of deuterium atoms in deuterated acrylic acid or deuterated methacrylic acid product}}{\text{Number of hydrogen atoms in acrylic acid or methacrylic acid starting material}} \times 100$$

$$\text{Conversion ratio} = \frac{\text{Number of moles of acrylic acid or methacrylic acid reaction product}}{\text{Number of moles of acrylic acid or methacrylic acid charger}} \times 100$$

Example 1

8.6 parts of methacrylic acid, 40 parts of heavy water, 0.3 parts of hexachloroiridate, and a trace of hydroquinone as the polymerization inhibitor were placed in a small autoclave and reacted for 24 hours at 110°C under stirring. After cooling, the reaction product was analyzed and found to be deuterated methacrylic acid with a conversion ratio of 100% and a deuteration ratio of 26%.

Example 2

The same process was carried out as in Example 1, except that the 0.3 parts of hexachloroiridate was replaced with 0.58 parts of tetrakis(triphenylphosphine)-palladium. This gave deuterated methacrylic acid with a conversion ratio of 100% and a deuteration ratio of 84%.

Example 3

7.2 parts of acrylic acid, 40 parts of heavy water, 1.4 parts of sodium hexachloroiridate, 22 parts of dimethyl acetamide, and a trace of hydroquinone were placed in a flask fitted with a condenser and reacted for 60 hours at 90°C under stirring. This gave deuterated acrylic acid with a conversion ratio of 79% and a deuteration ratio of 25%.

3

Examples 4-7

These reactions were carried out as in Example 1 except that as the catalyst there were used 0.38 parts of potassium bromoplatinate (Example 4), 0.2 parts of potassium pentachlororuthenate (Example 5), 0.23 parts of palladium nitrate (Example 6), 0.2 parts of potassium hexahydroxoplatinate (Example 7), and further reaction temperature and time were changed as shown in the following Table. The results are given in the following Table.

4

| Examples | Catalyst | Reaction Temp. (°C) | Time (hrs.) | Conversion Ratio (%) | Deuteration Ratio (%) |
|---|---|---|---|---|---|
| 4 | Potassium bromoplatinate | 100 | 24 | 70 | 37 |
| 5 | Potassium pentachlororuthenate | 100 | 24 | 82 | 34 |
| 6 | Palladium nitrate | 110 | 24 | 76 | 22 |
| 7 | Potassium hexahydroxoplatinate | 110 | 24 | 99 | 40 |

Example 8

4.3 parts of methacrylic acid, 40 parts of heavy water, 0.1 part of sodium hexachlororhodate, 0.1 part of potassium tetrachloroplatinate and a trace of hydroquinone as the polymerization inhibitor were placed into

5

a small autoclave and reacted for 24 hours at 100°C under stirring. This gave deuterated methacrylic acid with a conversion ratio of 100% and a deuteration ratio of 65%.

## Claims

1. A process for the production of deuterated acrylic acid or deuterated methacrylic acid comprising the direct deuteration of acrylic acid or methacrylic acid with heavy water or a mixture of heavy water and deuterium gas in the presence of a catalyst selected from compound(s) involving palladium, ruthenium, iridium and/or platinum used in an amount of at least 0.023 parts by weight per 1 part acid at a temperature up to 300°C.

2. A process according to claim 1, wherein a solvent stable at the reaction temperature is used as a co-solvent medium to heavy water.

3. A process according to claim 2, wherein dimethylacetamide or dimethylformamide is used as the co-solvent medium.

4. A process according to claim 1, wherein the reaction is carried out at a reaction temperature of from 60 to 200°C.

5. A process according to claim 1 wherein the deuteration is carried out in the presence of tetrakis-(triphenylphosphine)-palladium as a catalyst and without a co-solvent.

## Patentansprüche

1. Verfahren zur Herstellung von deuterierter Acrylsäure oder deuterierter Methacrylsäure, umfassend die direkte Deuterierung von Acrylsäure oder Methacrylsäure mit schwerem Wasser oder einem Gemisch aus schwerem Wasser und Deuteriumgas in Gegenwart eines Katalysators, der unter Verbindungen von Palladium, Ruthenium, Iridium und/oder Platin ausgewählt ist, in einer Menge von mindestens 0,023 Gewichtsteilen pro 1 Teil Säure bei einer Temperatur bis zu 300°C.

2. Verfahren nach Anspruch 1, wobei ein bei der Reaktionstemperatur stabiles Lösungsmittel als Kolösungsmittel für das schwere Wasser verwendet wird.

3. Verfahren nach Anspruch 2, wobei Dimethylacetamid oder Dimethylformamid als Kolösungsmedium verwendet werden.

4. Verfahren nach Anspruch 1, wobei die Umsetzung bei einer Reaktionstemperatur von 60 bis 200°C durchgeführt wird.

5. Verfahren nach Anspruch 1, wobei die Deuterierung in Gegenwart von Tetrakis-(triphenylphosphin)-palladium als Katalysator und ohne Kolösungsmittel durchgeführt wird.

## Revendications

1. Un procédé de production d'acide acrylique deutéré ou d'acide méthacrylique deutéré comprenant la deutération directe d'acide acrylique ou d'acide méthacrylique avec de l'eau lourde ou un mélange d'eau lourde et de deutérium gazeux en présence d'un catalyseur choisi parmi les composés contenant du palladium, du ruthénium, de l'iridium et/ou du platine, utilisé en une quantité d'au moins 0,023 partie en poids par partie d'acide à une température pouvant aller jusqu'à 300°C.

2. Un procédé selon la revendication 1, dans lequel on utilise un solvant stable à la température de réaction comme milieu co-solvant à l'eau lourde.

3. Un procédé selon la revendication 2, dans lequel on utilise du diméthylacétamide ou du diméthylformamide comme milieu co-solvant.

4. Un procédé selon la revendication 1, dans lequel on conduit la réaction à une température de réaction de 60° à 200°C.

5. Un procédé selon la revendication 1, dans lequel on effectue la deutération en présence de tétrakis-(triphénylphosphine)-palladium comme catalyseur et en l'absence de co-solvant.